# EUROPEAN PATENT APPLICATION

(11) **EP 1 435 392 A1**
(43) Date of publication of application: **07.07.2004**
(21) Application number: 02765541.4
(22) Date of filing: 13.09.2002
(51) Int. Cl.: C12P 41/00, C12R 1/685, C12R 1/67, C12R 1/66, C12R 1/785

(54) **PROCESS FOR PRODUCING D-ALANINE**

(30) Priority: 04.10.2001 JP 2001308785
(71) Applicant: Musashino Chemical Laborarory Ltd., Tokyo 104-0031 (JP)
(72) Inventor: UEDA, Hiroyuki, Tokyo 168-0081 (JP)
(74) Representative: DTS München
(86) International application number: PCT/JP2002/009436
(87) International publication number: WO 2003/031638

(57) **Abstract**

This invention is characterized by having filamentous fungi act on a DL-alanine containing solution and assimilate L-alanine without by-producing pyruvic acid. The D-alanine can be produced efficiently because the filamentous fungi can be conveniently and easily separated from the fermentation broth and the fermentation broth contains no pyruvic acid. For the purpose of isolating the D-alanine from the separating solution, it suffices to treat the separating solution by the use of an ion exchange resin. The filamentous fungi effectively usable herein belong to at least one member selected from the class consisting of genus Aspergillus, genus Penicillium, genus Mucor, genus Rhizopus, genus Circinella, genus Cladosporium, genus Trichoderma, genus Eurotium, genus Chaetomium, genus Aureobasidium, genus Geotrichum, and genus Paecilomyces which are commercially available.

## Description

### Technical Field

This invention relates to a method for producing D-alanine by having filamentous fungi assimilate the L-alanine contained in the DL-alanine.

### Background Art

D-alanine is an unnatural amino acid and this compound promises application as raw materials for pharmaceutical preparations and food additives. The method for producing D-alanine is known in various types including methods such as organic asymmetric synthesis, fermentation, and enzymological process which produce D-alanine from compounds other than D-alanine, a method of optical resolution by means of chromatography, a method of fractional crystallization of a racemic modification, and a method for exclusively recovering D-alanine by selective assimilation. The method of selective assimilation, on account of its ability to mass-produce D-alanine easily from an inexpensive DL-alanine as the raw material, has been enjoying a conspicuous development.

As one form of the method of selective assimilation, a method for producing optically active alanine and pyruvic acid by having a microorganism capable of oxidizing L-alanine form D-alanine and pyruvic acid from DL-alanine and then isolating these products is disclosed in the official gazette of JP-B 1967-20683.

The microorganisms capable of oxidizing L-alanine which are used in the invention include the strains belonging to such filamentous fungi and yeasts as Aspergillus niger, Penicillium chrysogenum, Candida tropicalis, and Crystococcus albidus saccharomius sake as well as Bacillus licheniformis, Pseudomonas aeruginosa, Corynebacterium oleophilus, Micrococcus conglomatas, and Pseudomonas cruciviae. The working examples cited therein obtain D-alanine and pyruvic acid by removing used cells from a fermentation broth by centrifugal separation and then treating the residue with an ion exchange resin.

The JP-B 1996-8878 discloses a method for collecting D-alanine by having a yeast assimilate L-alanine in a culture medium containing DL-alanine as a single carbon source and a single nitrogen source. This invention produces D-alanine at a cumulative concentration of not less than several tens of g/L by culturing a specific yeast in a culture medium of specific carbon source and a specific nitrogen source.

As a method of selective assimilation by a yeast, the official gazette of JP-A HEI-2-49598 discloses a method for producing D-alanine by effecting the assimilation by means of a microorganism capable of selectively decomposing only the L-alanine in the DL-alanine without forming pyruvic acid as an intermediate.

The method disclosed in the JP-B 1967-20683 is claimed to obtain D-alanine exclusively by having the filamentous fungi of Aspergillus niger, for example, assimilate L-alanine. Since it forms pyruvic acid at the same time, it necessitates an extract separating device for the purpose of separating D-alanine exclusively. Since the method of this official gazette particularly involves alkali neutralization, it suffers the by-produced pyruvic acid to coexist as an unsteady pyruvate and consequently give rise to a product of decomposition. Moreover, when the filamentous fungi are used, the cumulative concentration has such an extremely low magnitude as about 9 g/L.

Since the methods disclosed in the JP-B 1996-8878 and the JP-B 1990-49598 both use yeast, it is required such steps as precision filtration, ultrafiltration, and high-speed centrifugation in order to separat the yeast from the fermentation broth.

Generally, the cell size of yeast is in the approximate range of 1 - 5 µm. The method of filtration for use in solid-liquid separation, therefore, cannot be adopted for separating the yeast cells from the fermentation broth because the yeast cells pass the filter paper which is used for this method. Thus, the separation requires either treatment using a special separating membrane for precision filtration or ultrafiltration which has fine pore diameters or process for high-speed centrifugal separation. The method using a special separating membrane spends a longer time of treatment than the ordinary method of filtration because of a slower speed of permeation. When the special separating membrane is used in the fermentation broth to separate the cells therefrom, such components as fine suspended particles, proteins, and various microorganic metabolites as well as cells are deposited on the surface of the membrane to degrade the efficiency of filtration and decrease the service life of the expensive filtering membrane as well. The high speed centrifugal separator is expensive in itself and calls for a large power cost because the treatment therewith is performed at a high speed.

In these circumstances, the development of a method of selective assimilation which produces D-alanine easily in a high yield has been yearned for.

### Disclosure of the Invention

This invention, as a result of a deliberate study regarding methods for production of D-alanine by selective assimilation, has been perfected by a discovery that the use of a filamentous fungi permits exclusive assimilation of L-alanine without substantially by-producing pyruvic acid, moreover the use of the filamentous fungi enables specification of a culture medium, conditions for culture, and separation of cells to be greatly simplified, and the simplicity of the composition of a culture medium and the easiness of the separation of cells allow the target product to be obtained in a high purity by the subsequent process for purification of D-alanine. To be specific, this invention is directed toward providing a method for producing D-alanine, characterized by having a filamentous fungi act on a DL-alanine containing solution thereby inducing assimilation of L-alanine without substantially by-producing pyruvic acid.

According to this invention, D-alanine can be efficiently produced from DL-alanine by using a filamentous fungi. Particularly since the filamentous fungi separate cells easily, the separation of D-alanine as the target product can be easily separated. Further, since the assimilation of L-alanine does not entail by-production of pyruvic acid, the necessity for an operation of separating the product from D-alanine is obviated.

### Best Mode of Embodying the Invention

The first aspect of this invention consists in a method for producing D-alanine, characterized by having a filamentous fungi act on a DL-alanine containing solution thereby inducing assimilation of L-alanine without substantially by-producing pyruvic acid. This invention is characterized by havig the filamentous fungi act on the DL-alanine containing solution. Since the filamentous fungi manifests an only small auxotrophic property and moreover develops hyphae under culturing conditions as implied by its designation and, therefore, can be cultured in the form of pellets or lumps, the separation of cells contained in the fermentation broth can be effected comparatively easily. Further, since the assimilation entails substantially no by-production of pyruvic acid, the neutralization of the fermentation broth does not induce the presence of an unstable pyruvate, the subsequent separation of D-alanine is accomplished easily.

First, the DL-alanine serve as a substrate does not need to be restricted to the composition which has D-alanine and L-alanine contained in equal amounts. As long as it contains D-alanine as an essential requirement, any ratios of the two forms are available. Since the method of selective assimilation consists in assimilating L-alanine and consequently retaining D-alanine alone as a residue, the substrate to be used is preferred to contain D-alanine in a high ratio.

The variety of filamentous fungi advantageously usable in this invention are the species which belong to genus Aspergillus, genus Penicillium, genus Mucor, genus Rhizopus, genus Circinella, genus Cladosporium, genus Trichoderma, genus Eurotium, genus Chaetomium, genus Aureobasidium, genus Geotrichum, and genus Paecilomyces and which, when made to act on DL-alanine under specific fermentation conditions, assimilate L-alanine and avoid yielding pyruvic acid. As concrete examples of such preferable species of filamentous fungi, Aspergillus niger IAM 2561, Aspergillus tamarii IFO 4099, Circinella muscae IFO 4457, Cladosporium cladosporicides IFO 4459, Mucor racemosus f. sp. Racemosus IFO 4581, Trichoderma reesei IFO 3126, Rhizopus oryzae, Aspergillus flavus IFO 7599, OI-164, Penicillium chrysogenum IFO 4626, IFO 4640, IFO 6223, IFO 32030, Mucor ambiguus IFO 8092, and Mucor circinelloides IFO 4574 may be cited. This invention allows these species of filamentous fungi to be used either singly or in the form of a mixture of two or more members.

When such a species of filamentous fungi is caused to act on DL-alanine and consequently assimilate the L-alanine, the selection of a specific culture medium permits the D-alanine to be isolated without inducing by-production of pyruvic acid.

As the nutrient source for the filamentous fungi mentioned above, such assimilable nutrient sources in popular use as, for example, carbohydrates, nitrogen sources, and inorganic substances are available. As the carbon source, corn starch, corn meal, starch, dextrin, malt, glucose, glycerin, sucrose, and molasses may be used either singly or in the form of a mixture. As the nitrogen source, inorganic and organic nitrogen sources such as ammonium sulfate, sodium nitrate, soybean meal, corn steep liquor, gluten meal, meat extract, powdered fatty meat bone, yeast extract, dried yeast, cotton seed powder, peptone, polypeptone, wheat germ, fish meal, meat meal, defatted rice bran, powdered defatted meat bone, malt extract, and corn gluten meal may be used either singly or in the form of a mixture. As the inorganic salt, various inorganic salts such as calcium carbonate, sodium chloride, potassium chloride, magnesium sulfate, sodium bromide, sodium borate, primary potassium sulfate, zinc sulfate, and magnesium sulfate may be used either singly or in the form of a mixture. Optionally, such heavy metals as iron, manganese, zinc, cobalt, and molybdenum may be added in a very minute amount. The culture medium may incorporate additionally therein any of defoaming agents including vegetable oils such as soybean oil and linseed oil, higher alcohols such as octadecanol, and various silicones with the object of preventing the culture medium from generating foam during the course of thermal sterilization or in the process of culturing. The ratio of the nutrient source to the culture medium does not need to be particularly restricted but may be varied in a wide range. The optimum composition of the nutrient source and the ratio thereof to the culture medium can be easily decided in accordance with the conditions to be adopted by a simple small-scale experiment.

The culture medium to be used for the preculture in this invention, though variable with the species of filamentous fungi to be used and the conditions of the main culture to be adopted subsequently, is preferred to be a liquid culture medium containing a carbon source in the range of 0 - 100 g/L, preferably 20 - 80 g/L, a nitrogen source in the range of 0.5 - 50 g/L, preferably 1 - 15 g/L, and an inorganic substance in the range of 0.5 - 5 g/L, preferably 0.6 - 2 g/L.

The filamentous fungi propagated as described above are then transplanted to the culture medium for the main culture so as to produce D-alanine therein. This culture medium for the main culture has a composition such that the content of DL-alanine is in the range of 10 - 150 g/L, that of L-alanine containing nitrogen source in the range of 5.0 - 75 g/L, and that of inorganic salts in the range of 0.1 - 5 g/L. That is, while this invention, in the course of prepropagation, propagates the filamentous fungi in a culture medium incorporating saccharides therein, it is capable of producing D-alanine in the main culture using absolutely no saccharides in the culture medium. The use of saccharides is at a disadvantage in adding to cost and necessitating an extra step for the separation of saccharides. In contrast, this invention is capable of effecting the main culture with the composition mentioned above and, therefore, simplifying the process of purification, and reducing the cost required for the preparation of the culture medium as well. Especially, it is preferable to be carried out in a culture medium using the L-alanine substantially alone as a carbon source and a nitrogen source and additionally incorporating an inorganic salt in a small amount. Naturally, the culture medium for this main culture may additionally incorporate therein the nutrient source of the kind used in the prepropagation mentioned above. When such saccharum as glucose is used as the carbon source for the incorporation, it actually retards the assimilation of L-alanine because the growth of the filamentous fungi utilizes the saccharum prior to L-alanine. Further, it possibly renders the subsequent purification difficult because the metabolism of the saccharum by the microbe gives rise to a by-product and suffers the unassimilated saccharum to persist. Though the culture medium may additionally incorporate therein the nitrogen source of the kind prescribed for use in the prepropagation above, the nitrogen source is at a disadvantage in being generally expensive, giving a cause for coloring a liquid culture medium, and rendering the subsequent purification difficult. To be specific, the main culture is preferably carried out in a culture medium using the L-alanine substantially alone as a carbon source and a nitrogen source and additionally incorporating an inorganic salt in a small amount and optionally further incorporating a nutrient source in a small amount for the purpose of ensuring proper proceeding of the culture without inducing the problematic points mentioned above. By utilizing filamentous fungi which fit a specific culture medium as described above, pyruvic acid can not be formed as a by-product, or be lowered to the amount, it is made possible to obtain a liquid culture medium containing D-alanine substantially alone, simplify the process of purification, and decrease the expense required for the preparation of a culture medium to a lower level.

As the carbon source which can be incorporated in the culture medium for the main culture, cornstarch, starch, glucose, sucrose, and molasses may be used either singly or in the form of a combination of two or more members. As the nitrogen source, while L-alanine alone may be used, soy bean flour, corn steep liquor, gluten meal, meat extract, powdered fatty meat bone, yeast extract, dried yeast, peptone, polypeptone, and wheat germ may be used either singly or in the form of a combination of two or more members. The carbon source and the nitrogen source mentioned above do not need to be incorporated in the culture medium unconditionally. As concrete examples of the inorganic salt which is preferably incorporated in the culture medium, dihydrogen potassium phosphate and magnesium nitrate may be cited.

The culture medium for use in the main culture is preferred to have the pH value thereof adjusted prior to culture with an acid or an alkali so that it may fall in the range of 5 - 7 subsequent to sterilization.

The method for transplanting the spores of the filamentous fungi according to this invention does not need to be particularly restricted. Generally, a method which effects the transplantation by suspending the spores mentioned above in the following liquid and inoculating the resultant liquid suspension in an unmodified form to the liquid culture medium for use in fermentation may be used. As a means to suspend the spores mentioned above, a method which comprises growing filamentous fungi on an agar slant culture medium, adding the cells of the filamentous fungi which have consequently formed spores and a liquid together, and mixing them may be adopted. As the liquid mentioned above, sterile water is generally used. As concrete examples of the liquid which is used in the biochemical field, sterile waters incorporating therein Tween type surfactants such as Tween 80 (registered trademark), Triton type surfactants such as Triton X series (registered trademark), and acyl sorbitan each in a small amount (0.01 - 0.1 g/L of the whole liquid) may be cited. Thus, it is made possible to have the spores effectively dispersed in the sterile water and consequently obtain a homogeneous suspension.

The culture of the strain in the culture medium described above, in principle, can be performed by following with necessary modifications of the liquid culture which is generally employed in the production of D-amino acid. The liquid culture may be implemented in any of the known forms including stationary culture, spinner culture, shaking culture, and culture under aeration. This invention particularly prefers shaking culture and spinner culture under deep aeration. The culture proceeds properly under aeration. The amount of the air to be introduced to the culture medium falls preferably in the range of 0.1 - 2 vvm and more preferably 0.5 - 1.2 vvm. As the factor of satisfying this conditions, the culture device does not need to be particularly restricted. The culture is preferred to be performed with either of the stirring type reactor and the air lift type reactor while the culture medium is kept swept with air, pure oxygen, or the mixed gas thereof. Particularly, the air lift type reactor is excellent in respect that it avoids entangling the hyphae in the stirring vanes and prevents the hyphae themselves from breaking.

Further, as regards the operation of the culture, the culture may be performed continuously owing to the introduction of air or oxygen, batchwise by renewing a liquid culture medium for each batch, or semi-batchwise by feeding a liquid culture medium anew for each batch.

The DL-alanine as the substrate is only required to be supplied to the reaction solution in a concentration falling in the range of 20 - 400 g/L and preferably 40 - 200 g/L. It may be added wholly at the start of the reaction or piecemeal along the course of the reaction. At this time, the substrate concentration falls in the range of 10 - 150 g/L and preferably 20 - 100 g/L. If the substrate concentration falls short of 10 g/L, the shortage will result in lowering the efficiency of production of D-alanine. Conversely, if this concentration exceeds 150 g/L, the excess will possibly render the growth of filamentous fungi difficult.

The culturing temperature does not need to be particularly restricted but is only required to fall in the range in which the L-alanine is assimilated without substantially inhibiting the propagation of the strain. It falls generally in the range of 20 - 40°C and preferably in the range of 30 - 35°C.

The culturing time may be decided by synthetically taking into consideration such factors as the time-course change of the assimilation of L-alanine and the kind of the reactor in use. In the case of the filamentous fungi of genus Aspergillus, genus Penicillium, genus Mucor, genus Rhizopus, genus Circinella, genus Cladosporium, genus Trichoderma, genus Eurotium, genus Chaetomium, genus Aureobasidium, genus Geotrichum, and genus Paecilomyces mentioned above, the L-alanine can be assimilated fully satisfactory in a period falling in the range of 20 - 200 hours and preferably 40 - 120 hours. In the meantime, since the filamentous fungi even after a continuous operation incurs only a small reduction in the activity of assimilating L-alanine, it can be reused batchwise or semi-batchwise or used continuously. When they are continuously used, the culturing time may be properly adjusted by periodically checking their activity of assimilating L-alanine along the course of time.

In this invention, a D-alanine containing solution can be obtained by having filamentous fungi act on a DL-alanine containing solution and assimilate L-alanine without substantially by-producing pyruvic acid. Meanwhile, in consequence of the assimilation of L-alanine, the L-alanine is decomposed into ammonia, carbon dioxide, oxalic acid, water, etc. Thus, the pH value of the culture medium is controlled in the neighborhood of neutrality which fits the assimilation of L-alanine by the filamentous fungi, specifically in the neighborhood of 4 - 8, and preferably 5 - 7 by performing the culture while keeping the ammonia generated by the assimilation neutralized with an acid and keeping the oxalic acid generated by the assimilation neutralized with an alkali. This treatment of neutralizing the by-products is substantially materialized owing to the fact that the filamentous fungi used in this invention do not by-produce pyruvic acid. That is, pyruvic acid, as clearly inferred from the chemical structure thereof, is a compound which is very easily decomposed by decarboxylation. When pyruvic acid is formed in a culture medium, the neutralizing treatment performed on other by-products affects pyruvic acid at the same time and consequently makes it induce the coexistence of ammonia and oxalates. It is because the pyruvates are so liable to be decomposed as to yield to decomposition during the treatment of by-products that the by-products cannot be separated substantially from the fermentation broth. Specifically, owing to the use of filamentous fungi which are incapable of by-producing pyruvic acid, this invention is enabled to facilitate the subsequent process of purifying D-alanine and exalt the yield of the process. The expression "incapable of substantially by-producing" as used in the specification of this invention means that absolutely no pyruvic acid is by-produced or pyruvic acid is by-produced in an amount of not more than 0.1 mol% relative to the amount of alanine.

As the acid which can be used for the purpose of neutralizing ammonia, sulfuric acid, hydrochloric acid, nitric acid, acetic acid, oxalic acid, phosphoric acid, etc. are available and sulfuric acid and phosphoric acid are particularly preferably usable. Sulfuric acid and ammonia generate ammonium sulfate and this salt can be removed with ion exchange resin or by electrodialysis. When an acid is to be added, it suffices to prepare an aqueous solution containing the acid in a concentration adjusted in the range of 2.5 - 25 mass %, preferably 5 - 10 mass %, and add dropwise to a given culture medium this aqueous solution in an amount of neutralization to be decided by determining the amount of ammonia contained in the culture medium. Optionally, the culture medium may be automatically maintained at a proper pH value by effecting the addition with the aid of a pH sensor or a pH controller during the course of the culture.

As the alkali which is used for the purpose of neutralizing a by-produced oxalic acid, calcium carbonate, sodium hydroxide, aqueous ammonia, and ammonia gas are available and aqueous ammonia and sodium hydroxide are particularly preferably usable. Optionally for this purpose, the culture medium may be automatically maintained at a proper pH value by adding calcium carbonate to the culture medium prior to having the culture medium sterilized or adding an aqueous sodium hydroxide solution, aqueous ammonia, or ammonia gas through the medium of a pH sensor or a pH controller during the course of the culture. When the kind of filamentous fungi is such that ammonia is by-produced simultaneously with oxalic acid, since the ammonia neutralizes the oxalic acid, it suffices to neutralize the oxalic acid by adding ammonia in an amount enough to fill short supply.

The term "neutralize" used in this invention refers to an operation of adding an acid to ammonia or adding an alkali to oxalic acid so as to decrease the alkalinity or acidity and does not necessarily mean to adjust the pH value thereof to 7. By the operation of neutralizing mentioned above, the pH value of the culture medium is controlled to a range of 4 - 8, preferably 5 - 7. By this control, it is made possible to adapt the culture medium for the assimilation of L-alanine by the filamentous fungi to be used and separate the by-products from D-alanine.

This invention separates the cells from the fermentation broth by adopting any of the known methods for separating cells as by filtration, centrifugal separation, and filter pressing, for example. Among other methods enumerated above, the filtration is convenient and advantageous for the separation of hyphae and cells suspended on the fermentation broth.

In this invention, by having filamentous fungi act on the DL-alanine, it is made possible to decompose L-alanine into ammonia, carbon dioxide, oxalic acid, and water and meantime avoid substantially by-producing pyruvic acid as described above. Thus, the separating solution which has removed the cells from the culture broth contains D-alanine as a main component and barely contains amino acids which have been used in the preparation of the culture medium and are now remaining as decomposed in a minute amount. It, therefore, eminently facilitates the subsequent purification and isolation of D-alanine. Incidentally, this easiness of the separation and purification does not mean the easiness of the separation merely from pyruvic acid. Pyruvic acid, as described above, is very unstable as in heat and easily succumbs to decomposition with generation of carbon dioxide. When the assimilation of L-alanine and the by-production of pyruvic acid simultaneously occur, it becomes necessary to set the conditions for the separation of pyruvic acid and pyruvates and further select mild conditions incapable of decomposing the compounds so separated. Otherwise, it becomes necessary to perform an operation for separating the compounds resulting from the decomposition. When ammonia is a sole by-product, for example, the ammonia can be removed in the form of a gas by heating the separating solution. When pyruvic acid is additionally by-produced, however, the heat treatment just mentioned cannot be effectively performed. When the separation is effected by the use of an ion exchange resin as a means of avoiding application of heat, the pyruvic acid decomposes in the resin, induces a change in the pH value, and degrades the ability to separate from D-alanine. In this invention, however, since the culture medium is prevented from containing pyruvic acid, such degradation of the ability of the ion exchange resin to separate D-alanine as mentioned above is suffered to occur only to a minute degree. Specifically, it is because this invention allows no by-production of pyruvic acid that the separation of D-alanine from the separating solution resulting from the separation of cells and containing D-alanine as a main component is made very simple and easy and the production of D-alanine is attained in a high yield.

In this invention, the isolation of D-alanine from the separating solution which has removed the cells can be effected by the electrodialysis method, the ion exchange resin method, the ion exchanging method·calcium oxalate·calcium sulfate precipitation removal method, etc. and the isolation of D-alanine can be effected particularly efficiently by the electrodialysis method or the ion exchange resin method. In this invention, the D-alanine can be exclusively isolated by the electrodialysis, ie by causing the isolated D-alanine to be retained in the dialysis membrane, because the D-alanine forms the main component of the amino acids contained in the separating solution.

When an ion exchange resin is to be used for the purpose of isolating the D-alanine from the separating solution, it is proper to adopt particularly an ammonia type ion exchange resin, cause this ion exchange resin to adsorb the D-alanine, and thereafter elute the adsorbate with ammonia. This is because this particular ion exchange resin is capable of simultaneously isolating the D-alanine and reverting the resin to the ammonia type and consequently enabling the used ion exchange resin to be easily put to reuse. As preferred ion exchange resins, "Diaion SK1B" made by Mitsubishi Kagaku K.K., "Amberlite IR-120B" made by Organo K.K., and "DOWEX HCR" and "DOWEX HGR" made by the Dow Chemical Company may be cited.

To be specific, the same fermentation broth as used in the treatment by electrodialysis is adjusted to pH 1 by the addition of concentrated sulfuric acid. Separately, the ion exchange resin packed in a column made of glass is converted to the NH⁴⁺ type by passing aqueous ammonia therethrough and then washed with deionized water. The amount of the resin is so adjusted that the molar ratio of exchange group/alanine may fall in the range of 1 - 6, preferably 2 - 5. The fermentation broth is passed through the ion exchange resin in the glass column. The space velocity of the flow of the fermentation broth falls preferably in the range of 0.2 - 5, and more preferably in the range of 0.4 - 4. The column, after passing the fermentation broth therethrough, passes water to elute sulfuric acid. Next, the column passes an aqueous 1 mole ammonia solution therethrough to elute the alanine adsorbed on the resin. By passing ammonia in an excess amount through the column, the resin can be reverted to the NH⁴⁺ type. Incidentally, the ammonia contained in the eluting solution can be removed by application of heat.

The oxalate which is contained in the separating solution after the removal of cells exhibits a low degree of solubility to water. Thus, the separation may be accomplished by the method of utilizing the difference in the degree of solubility between the oxalate and the D-alanine. To be more specific, the separation of the oxalate can be attained by inducing precipitation of the oxalate. The quantitative analysis of D-alanine and L-alanine was carried out by HPLC.

### Examples

Now, this invention will be described below with reference to working examples. This invention is not restricted by these examples. The symbol "%" denotes "% by mass." The conditions for the analysis of D,L-alanine by HPLC were: column: "OA-6100" made by Sumika Bunseki Center, column diameter x column length: 4.6 mmϕ x 150 mm, eluent: aqueous 1 mM copper sulfate solution, flow rate: 1 ml/min, Detector: UV detector, wavelength: 254 nm.

### [Examples 1 - 5]

A preculture medium of a composition shown in Table 1 was dispensed in Erlenmeyer flasks having a prescribed inner volume and sterilized in an autoclave at 121°C for 15 minutes to obtain aliquots of preculture medium. A strain in a prescribed amount shown in Table 2 was inoculated to the aliquots with a platinum loop or in the form of a spore suspension and cultured with a rotary shaker under the conditions shown in Table 2. After the preculture, the precultured fermentation broth was transplanted in a prescribed amount shown in Table 4 to a sterilized main culture medium (held in a Sakaguchi flask) and subjected to flask culture. The filamentous fungi shown in Table 4 were used in this experiment. After the culture was completed, the fermentation broth was centrifuged at 3600 rpm for 10 minutes to remove the cells. The supernatant consequently formed was diluted to a prescribed concentration and analyzed by HPLC to determine the residual amounts of L form and D form.

The compositions of the individual preculture media are shown in Table 1, the conditions for the preculture in Table 2, the compositions of the main culture media in Table 3, and the conditions for the main culture in Table 4. The results of the analysis of the relevant separating solutions are shown in Table 5. In Table 5, the numerals in the left column indicate Examples 1 - 5, the numerical values of L form and D form indicate the masses (g/L) remaining in the culture media, and the column of culture indicates the duration of culture (hr).

**Table 1**

| Preculture media | | | | | |
|---|---|---|---|---|---|
| Composition of culture medium (g/L) | Example 1 | Example 2 | Example 3 | Example 4 | Example 5 |
| Starch | 10 | - | - | - | 50 |
| Polypeptone | - | 5 | 10 | 10 | 5 |
| Glucose | 10 | - | 2 | 2 | 10 |
| Beef extract | - | 10 | 30 | 30 | - |
| (NH₄)₂SO₄ | 1.35 | - | - | - | - |
| KH₂PO₄ | 0.3 | 1 | 1 | 1 | 1 |
| MgSO₄·7H₂O | 0.25 | 0.5 | 0.5 | 0.5 | 0.5 |
| ZnSO₄·7H₂O | 0.04 | - | - | - | - |
| DL-alanine | - | 2 | - | - | - |
| pH | No adjustment | No adjustment | No adjustment | No adjustment | No adjustment |

**Table 2**

| Conditions for preculture | | | | | |
|---|---|---|---|---|---|
| | Example 1 | Example 2 | Example 3 | Example 4 | Example 5 |
| Amount of transplanted fungi (Number of spores/ml-culture medium) | 2 x 10⁶ pieces | 2 x 10⁶ pieces | One platinum loop full | One platinum loop full | 10⁶ pieces |
| Culturing temperature (°C) | 30 | 30 | 30 | 30 | 30 |
| Rotational velocity (rpm) | 220 | 220 | 210 | 220 | 220 |
| Culturing time (h) | 21 | 21 | 21 | 21 | 21 |
| Amount charged (ml) | 30 | 30 | 50 | 50 | 50 |
| Inner volume of culturing vessel | 200 ml | 200 ml | 300 ml | 300 ml | 300 ml |

**Table 3**

| Main culture medium | | | | | |
|---|---|---|---|---|---|
| Composition of culture medium (g/L) | Example 1 | Example 2 | Example 3 | Example 4 | Example 5 |
| DL-alanine | 20 | 20 | 20 | 20 | 20 |
| Yeast extract | 0.5 | 0.5 | 0.5 | 0.5 | 0.5 |
| KH₂PO₄ | 1 | 1 | 1 | 1 | 1 |
| MgSO₄·7H₂O | 0.5 | 0.5 | 0.5 | 0.5 | 0.5 |
| PH | 5 | 5 | 5 | 5 | 5 |

**Table 4**

| Conditions for main culture | | | | | |
|---|---|---|---|---|---|
| | Example 1 | Example 2 | Example 3 | Example 4 | Example 5 |
| Amount of transplanted fungi (v/v%) | 5 | 5 | 10 | 10 | 10 |
| Culturing temperature (°C) | 30 | 30 | 30 | 30 | 30 |
| Rotational frequency (rpm) | 107 | 107 | 108 | 108 | 110 |
| Amount charged (ml) | 200 | 200 | 50 | 50 | 50 |
| Inner volume of culturing vessel | 500 ml | 500 ml | 500 ml | 500 ml | 500 ml |

**Table 5**

| | Filamentous fungi | L form | D form | Culture |
|---|---|---|---|---|
| 1 | *Rhizopus oryzae* MK9601 | 4.3 | 9.6 | 30 |
| 2 | *Rhizopus oryzae* MK9601 | 2.2 | 9.5 | 30 |
| 3 | *Aspergillus oryzae* IFO 4254 | 5.6 | 9.8 | 30 |
| | *Aspergillus oryzae var oryzae* IFO 6215 | 5.1 | 9.9 | 30 |
| | *Aspergillus parasiticus* IFO 4239 | 1.8 | 9.0 | 30 |
| | *Aspergillus candidus* IFO 4310 | 5.2 | 9.8 | 30 |
| | *Aspergillus flavus* IFO 7599 | 3.9 | 9.8 | 30 |
| | *Aspergillus flavus* OI-164 | 0.0 | 7.0 | 30 |
| | *Aspergillus niger van Tieghem* IAM 2561 | 0.0 | 9.5 | 30 |
| | *Aspergillus terreus* IFO 6365 | 5.7 | 9.9 | 30 |
| | *Mucor circinelloides* IFO 4574 | 1.7 | 8.0 | 30 |
| | *Mucor ambiguus* IFO 8092 | 0.1 | 7.6 | 30 |
| | *Mucor dimorphosporus* IFO 4555 | 3.5 | 9.6 | 30 |
| | *Mucor dimorphosporus* IFO 4556 | 6.0 | 10.0 | 30 |
| 4 | *Aspergillus niger* IFO 4066 | 8.4 | 9.7 | 30 |
| | *Aspergillus niger* IFO 4067 | 0.8 | 9.6 | 30 |
| | *Aspergillus niger* IFO 4068 | 3.6 | 9.8 | 30 |
| | *Aspergillus niger* IFO 4043 | 5.0 | 9.8 | 30 |
| | *Aspergillus niger* IFO 4281 | 6.3 | 9.7 | 30 |
| | *Penicillium chrysogenum* IFO 4626 | 3.5 | 9.6 | 30 |
| | *Penicillium chrysogenum* IFO 4640 | 3.6 | 8.3 | 30 |
| | *Penicillium chrysogenum* IFO 6223 | 3.2 | 9.3 | 30 |
| | *Penicillium chrysogenum* IFO 32030 | 8.3 | 9.6 | 30 |
| | *Aspergillus niger* IAM 2561 | 0.1 | 9.0 | 30 |
| 5 | *Aspergillus niger* IAM 2561 | 3.7 | 9.7 | 30 |
| | *Eurotium repens* IFO 4041 | 8.1 | 9.8 | 30 |
| | *Aspergillus tamarii* IFO 4099 | 5.2 | 9.6 | 30 |
| | *Chaetomium globosum* IFO 4451 | 9.2 | 9.7 | 30 |
| | *Circinella muscae* IFO 4457 | 0.6 | 9.6 | 30 |
| | *Cladosporium cladosporioides* IFO 4459 | 7.6 | 9.6 | 30 |
| | *Mucor racemosus f. sp. Racemosus* IFO 4581 | 5.9 | 9.5 | 30 |
| | *Geotrichum candidum* IFO 4597 | 3.2 | 7.9 | 30 |
| | *Paecilomyces variotii* IFO 4855 | 8.4 | 9.5 | 30 |
| | *Trichoderma viride* IFO 5720 | 5.5 | 9.2 | 30 |
| | *Penicillium funiculosum* IFO 31132 | 8.8 | 9.5 | 30 |
| | *Trichoderma reesei* IFO 31326 | 3.9 | 9.3 | 30 |
| | *Aureobasidium microstictum* IFO 32065 | 7.7 | 9.3 | 30 |

### (Examples 6 - 9)

All the species of fungi used in Examples 1 - 5 were manipulated for separation of spores in accordance with a common method. The strains exhibiting excellent activity among other strains were selected. These selected strains were test cultured by the use of a jar fermenter having an inner volume of 3 L.

A preculture medium having a composition shown in Table 6 was dispensed in Erlenmeyer flasks having a prescribed inner volume and sterilized in an autoclave at 121°C for 15 minutes to obtain aliquots of preculture medium. Spores of an amount shown in Table 7 were transplanted to each of the aliquots and cultured by a rotary shaker under the conditions shown in Table 7. After the preculture, the preculture fermentation broth was transplanted in a prescribed amount to a jar fermenter having an inner volume of 3 liters which was charged with a main culture medium shown in Table 8 and then sterilized and was subjected to aerated spinner culture under the conditions shown in Table 9. The strains of filamentous fungi used herein are shown in Table 10. The main culture was carried out with the pH value of the medium adjusted by adding an aqueous 10% sulfuric acid solution to the formed ammonia and an aqueous 4 wt% ammonia solution to the by-produced oxalic acid. After the completion of the culture, the resultant fermentation broth was centrifuged at 3600 rpm for 10 minutes to remove the cells. The supernatant consequently formed was diluted to a prescribed concentration and then analyzed by HPLC to determine the residual amounts of L form and D form.

The compositions of the preculture media are shown in Table 6, the conditions for the preculture in Table 7, the compositions of the main culture media in Table 8, and the conditions for the main culture in Table 9. In Table 10, the numerals in the left column indicate Examples 6 - 9, the numerical values of L form and D form indicate the masses (g/L) remaining in the culture media, and the column of culture indicates the duration of culture (h).

**Table 6**

| Preculture medium | | | | |
|---|---|---|---|---|
| Composition of culture medium (g/L) | Example 6 | Example 7 | Example 8 | Example 9 |
| Starch | - | 50 | 50 | 50 |
| Polypeptone | 5 | 5 | 5 | 5 |
| Glucose | - | 10 | 10 | 10 |
| Beef extract | 10 | - | - | - |
| (NH₄)₂SO₄ | - | - | - | - |
| KH₂PO₄ | 1 | 1 | 1 | 1 |
| MgSO₄·7H₂O | 0.5 | 0.5 | 0.5 | 0.5 |
| ZnSO₄·7H₂O | - | - | - | 10 |
| CaCO₃ | | 10 (A. Niger alone added) | 10 (A. Niger alone added) | 10 |
| DL-alanine | 2 | - | - | - |
| PH | No adjustment | 6 | 6 | 6 |

**Table 7**

| Conditions for preculture | | | | |
|---|---|---|---|---|
| | Example 6 | Example 7 | Example 8 | Example 9 |
| Amount of transplanted fungi (Number of spores/ml - culture medium) | 2 x 10⁶ pieces | 10⁶ pieces | 10⁶ pieces | 10⁶ pieces |
| Culturing temperature (°C) | 30 | 30 | 30 | 30 |
| Rotational velocity (rpm) | 220 | 220 | 220 | 220 |
| Culturing time (h) | 21 | 18-21 | 21 | 21 |
| Amount charged (ml) | 30 | 110 | 110 | 110 |
| Inner volume of culturing vessel | 200 ml | 500 ml | 500 ml | 500 ml |

**Table 8**

| Main culture medium | | | | |
|---|---|---|---|---|
| Composition of culture medium (g/L) | Example 6 | Example 7 | Example 8 | Example 9 |
| DL-alanine | 50 | 50 | 100 | 100 |
| Yeast extract | 0.5 | 0.5 | 0.5 | 0.5 |
| KH₂PO₄ | 1 | 1 | 1 | 1 |
| MgSO₄·7H₂O | 0.5 | 0.5 | 0.5 | 0.5 |
| Defoaming agent (ml/2L of culture medium) | 1 | 1 | 1 | 1 |
| PH | | 5.5 | 5.5 (A.nige r:pH5) | 5-5.5 |

**Table 9**

| Condition of main culuture | | | | |
|---|---|---|---|---|
| | Example 6 | Example 7 | Example 8 | Example 9 |
| Amount of transplanted fungi (v/v%) | 5 | 5 | 5 | 5 |
| Culturing temperature (°C) | 30 | 30 | 30 | 30 |
| Rate of agitation (rpm) | 500 | 400-500 | 400-500 | 400-500 |
| Amount of aeration (vvm) | 1 | 1 | 1 | 1 |
| Amount charged (ml) | 2 | 2 | 2 | 2 |
| Inner volume of culturing vessel | 3L jar | 3L jar | 3L jar | 3L jar |

**Table 10**

| | Filamentous fungi | L form | D form | Culture |
|---|---|---|---|---|
| 6 | Rhizopus oryzae MK9601 | 14.8 | 24.0 | 22 |
| 7 | Penicillium chrysogenum IFO 4626 | 0.0 | 23.3 | 40 |
| | Aspergillus tamarii IFO 4099 | 0.4 | 20.9 | 40 |
| | Aspergillus niger IAM 2561 | 0.0 | 23.2 | 40 |
| | Aspergillus tamarii IFO 4099 | 0.0 | 20.9 | 40 |
| | Circinella muscae IFO 4457 | 15.7 | 23.2 | 40 |
| | Cladosporium cladosporioides IFO 4459 | 22.4 | 25.0 | 40 |
| | Mucor racemosus f. sp. Racemosus IFO 4581 | 21.5 | 24.3 | 40 |
| | Paecilomyces variotii IFO 4855 | 22.1 | 23.8 | 40 |
| | Trichoderma reesei IFO 31326 | 10.5 | 21.5 | 40 |
| 8 | Penicillium chrysogenum IFO 4626 | 1.0 | 45.1 | 90 |
| | Aspergillus tamarii IFO 4099 | 0.0 | 46.5 | 90 |
| | Penicillium chrysogenum IFO 32030 | 30.9 | 47.7 | 100 |
| | Penicillium funiculosum IFO 31132 | 42.6 | 45.2 | 100 |
| | Aspergillus niger IAM 2561 | 0.0 | 45.7 | 90 |
| 9 | Aspergillus niger IFO 4067 | 10.2 | 44.2 | 100 |
| | Aspergillus niger IFO 4068 | 10.5 | 42.0 | 100 |
| | Aspergillus niger IFO 4281 | 0.3 | 45.0 | 90 |

### (Example 10)

The volume 110 ml of a culture medium (pH 6.0) containing 50 g/L of starch, 10 g/L of glucose, 5 g/L of polypeptone, 1 g/L of dihydrogen potassium phosphate, and 0.5 g/L of magnesium sulfate heptahydrate was dispensed in Erlenmeyer flasks having an inner volume of 500 ml and sterilized in an autoclave at 121°C for 15 minutes to obtain a preculture medium. Penicillium chrysogenum IFO 4626 was transplanted to the preculture medium at a spore concentration of 1 x 10⁶ pieces/mL-clture medium and cultured by the use of a rotary shaker at 30°C at 220 rpm for 21 hours. Separately, a minijar fermenter having an inner volume of 3 L was charged with 2 L of a culture medium containing 150 g/L of DL-alanine, 1 g/L of yeast extract, 1 g/L of polypeptone, 1 g/L of dihydrogen potassium phosphate, 1 g/L of magnesium sulfate heptahydrate, and 0.5 ml/L of a defoaming agent and the contents of the fermenter were sterilized in an autoclave at 121°C for 15 minutes to obtain a main culture medium. The main culture medium and the former preculture medium transplanted thereto at a ratio of 5% were subjected together to aerated spinner culture at 30°C at 1.0 vvm. During the course of the main culture, the combined culture media were kept adjusted to pH 5.5 by the addition of an aqueous 10% sulfuric acid solution. In 120 hours, the whole amount of L-alanine in the culture medium was assimilated to obtain 2.2 L of a fermentation broth containing 149 g of D-alanine and 81 g of ammonium sulfate.

The fermentation broth was filtered through a filter paper containing pores 7.0 µm in diameter (made by Kiriyama Seisakusho K.K. and sold under the trademark designation of "Filter Paper No. 5A") to remove the cells. The solution obtained by the filtration was adjusted to pH 1 by the addition of sulfuric acid and then passed through a column packed with an ion exchange resin (made by Mitsubishi Kagaku K.K. and sold under the trademark designation of "Diaion SK-1B (MH4 + type)") to induce adsorption of D-alanine to the ion exchange resin. The column was thoroughly washed with water and then made to pass an aqueous 2% ammonia solution, with the result the D-alanine was adsorbed on the resin. The eluate was decolorized with activated carbon and concentrated till crystallization to give rise to 147 g of purified D-alanine. The D-alanine thus obtained had an optical purity of not less than 99.9%ee and a chemical purity of not less than 99.9%.

### (Example 11)

The fermentation broth which had undergone culture and removal of cells under the same conditions as used in Example 10 was adjusted to pH 6.0, ie the isoelectric point of alanine by the addition of an aqueous 28% ammonia solution and then subjected to electrodialysis till the electroconductivity thereof was lowered amply. At this point, the liquid in the sample room was recovered to obtain an aqueous D-alanine solution. This solution was decolorized with activated carbon and then concentrated till crystallization to obtain 145 g of purified D-alanine. The D-alanine thus obtained had an optical purity of not less than 99.9%ee and a chemical purity of not less than 99.9%.

### Industrial Applicability

This invention concerns a method for producing D-alanine by having a filamentous fungi assimilate the L-alanine contained in DL-alanine. The filamentous fungi can be separated conveniently and easily from the fermentation broth. The D-alanine can be produced efficiently because the fermentation broth contains no pyruvic acid.

## Claims

1. A method for producing D-alanine, **characterized by** having filamentous fungi act on a DL-alanine containing solution and assimilate L-alanine without substantially by-producing pyruvic acid.

2. A method for producing D-alanine, comprising a step of having filamentous fungi act on a DL-alanine containing solution, assimilate L-alanine without substantially by-producing pyruvic acid, and obtain a D-alanine containing solution, a step of separating the filamentous fungi contained in said D-alanine containing solution and obtaining a separating solution, and a step of isolating the D-alanine from said separating solution.

3. A method according to claim 2, wherein the step of obtaining said D-alanine containing solution is carried out while the ammonia generated by the assimilation is kept neutralized with an acid or while the oxalic acid formed by the assimilation is kept neutralized with an alkali.

4. A method according to claim 2 or claim 3, wherein the step of isolation D-alanine from the separating solution is effected by electrodialysis or by the use of an ion exchange resin.

5. A method according to claim 4, wherein said ion exchange resin is an ammonia type cation exchange resin.

6. A method according to any of claims 1 - 5, wherein said filamentous fungi belong to at least one member selected from the class consisting of genus Aspergillus, genus Penicillium, genus Mucor, genus Rhizopus, genus Circinella, genus Cladosporium, genus Trichoderma, genus Eurotium, genus Chaetomium, genus Aureobasidium, genus Geotrichum, and genus Paecilomyces.

7. A method according to any of claims 1 - 6, wherein said filamentous fungi belong to at least one member selected from the class consisting of Aspergillus niger, Aspergillus tamarii, Aspergillus flavus, Penicillium chrysogenum, Mucor ambiguus, Mucor circinelloides, and Cricinella muscae.
